# EUROPEAN PATENT APPLICATION

(11) **EP 2 062 892 A1**
(43) Date of publication of application: **27.05.2009**
(21) Application number: 07806912.7
(22) Date of filing: 07.09.2007
(51) Int. Cl.: C07D 471/04, A23K 1/16, A61K 31/437, A61P 9/12, A23L 1/30

(54) **HYPERTENSION-AMELIORATING AGENT**

(30) Priority: 08.09.2006 JP 2006244445
(71) Applicant: Kyowa Hakko Bio Co., Ltd., Tokyo 100-8185 (JP); Mitsubishi Gas Chemical Company, Inc., Chiyoda-ku Tokyo 100-8324 (JP)
(72) Inventor: OGINO, Fumiko, Tsukuba-shi Ibaraki 305-0841 (JP); KAMIYA, Toshikazu, Tsukuba-shi Ibaraki 305-0841 (JP); NAKANO, Masahiko, Niigata-shi Niigata 950-3112 (JP); KIKKAWA, Kazutoshi, Tokyo 100-8324 (JP)
(74) Representative: Vossius & Partner
(86) International application number: PCT/JP2007/067471
(87) International publication number: WO 2008/029907

(57) **Abstract**

It is an objective of the present invention to provide a safe and highly effective hypertension-ameliorating agent.

The present invention provides a hypertension-ameliorating agent, comprising a compound represented by general formula (I) 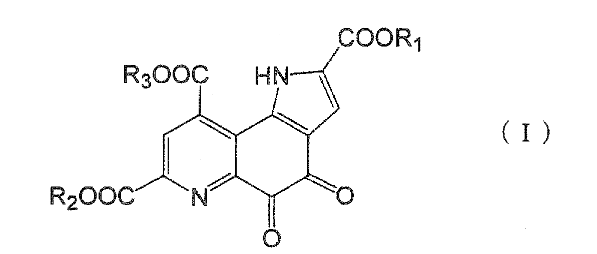 (wherein R₁, R₂, and R₃ are the same as or different from each other, and each represents lower alkyl, lower alkenyl, lower alkynyl, aralkyl, araryl, phenyl, or a hydrogen atom) or a salt thereof as an active ingredient.

## Description

### Technical Field

The present invention relates to a hypertension-ameliorating agent comprising pyrroloquinoline quinone or an ester or salt thereof as an active ingredient.

### Background Art

Hypertension is a risk factor that causes development of myocardial infarction, angina pectoris, cerebral infarction, cerebral hemorrhage, dementia caused by cerebrovascular disorders, or the like. Therefore, it is important to keep blood pressure within the normal range for health maintenance. Examples of hypertension therapeutic agents include: diuretics capable of inducing sodium and water diuresis that result in reduction in the circulating blood volume so as to cause a decrease in blood pressure; sympathetic nerve suppressors capable of centrally or peripherally suppressing the activity of the sympathetic nerve system that plays a very important role for vasoregulation so as to cause a decrease in blood pressure; calcium antagonists capable of suppressing incorporation of calcium into vascular smooth muscle cells so as to dilate blood vessels; and angiotensin I conversion enzyme (ACE) inhibitors capable of suppressing production of angiotensin II serving as a vasopressor and causing increases in the bradykinin and prostaglandin amounts so as to dilate blood vessels ("NEW YAKURIGAKU (pharmacology)" edited by Chikako Tanaka and Ryuichi Kato, the 3rd revised edition, Nankodo Co., Ltd., 1996, pp. 380-381).

Pyrroloquinoline quinone (hereinafter, abbreviated as "PQQ") was discovered in 1979 as a coenzyme of methanol dehydrogenase in methanol assimilating microorganisms (see "Nature," 1979, Vol. 230, pp. 843-844; and "FEBS Letters," 1979, Vol. 108, pp. 443-446). Other than such microorganisms, PQQ has also been detected in edible plants such as soybean, horse bean, green pepper, potato, parsley, and spinach and processed foods such as vinegar, tea, cocoa, natto, and tofu (see "Biochemical Journal," 1995, Vol. 307, pp. 331-333). Furthermore, the presence of PQQ in humans and rats *in vivo* has been reported (see "Biochimica et Biophysica Acta," 1992, Vol. 1156, pp. 62-66).

Hitherto, it has been revealed that PQQs have a variety of effects as follows: a cell growth promoting effect (see JP Patent Publication (Kokai) No. 61-58584 A (1986)), an active oxygen eliminating effect (see JP Patent Publication (Kokai) No. 5-078247 A (1993)), an aldose reductase-inhibiting effect (see JP Patent Publication (Kokai) No. 6-256191 A (1994)), a nerve growth factor production promoting effect (see JP Patent Publication (Kokai) No. 6-211660 A (1994)), a reverse transcriptase inhibiting effect (see JP Patent Publication (Kokoku) No. 8-005792 B (1996)), an anti-cataract effect (see JP Patent Publication (Kokoku) No. 8-005791 B (1996)), melanin production suppressing and skin lightening effects (see JP Patent Publication (Kokai) No. 8-020512 A (1996)), and an ultraviolet absorption effect (see JP Patent No. 3625493), for example.

However, it has not been known that PQQ or an ester or salt thereof has hypertension-ameliorating effects.

### Disclosure of the Invention

### Problem to be Solved by the Invention

It is an objective of the present invention to provide a hypertension-ameliorating agent.

### Means for Solving Problem

The present invention provides a hypertension-ameliorating agent as in the following (1) to (3).
(1) A hypertension-ameliorating agent, which comprises a compound [hereinafter, referred to as compound (I)] represented by general formula (I) 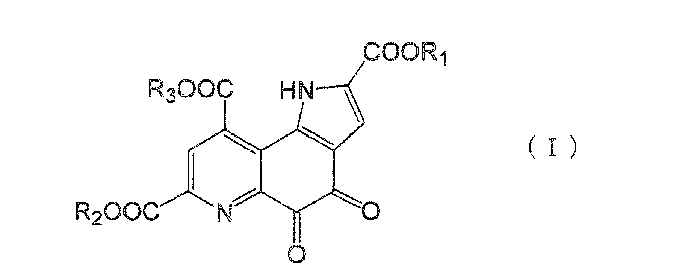 (wherein R₁, R₂, and R₃ are the same as or different from each other, and each represents lower alkyl, lower alkenyl, lower alkynyl, aralkyl, araryl, phenyl, or a hydrogen atom) or a salt thereof as an active ingredient.
(2) A method for improving hypertension, which comprises administering an effective amount of the compound represented by general formula (I) according to (1) above or a salt thereof to a subject in need thereof.
(3) Use of the compound represented by general formula (I) according to (1) above or a salt thereof for the manufacture of a hypertension-ameliorating agent.

### Effects of the Invention

The present invention provides a safe and effective hypertension-ameliorating agent.

This description includes part or all of the contents as disclosed in the description and/or drawings of Japanese Patent Application No. 2006-244445, which is the priority application of the present application.

### Best Mode for Carrying Out the Invention

According to the definition for compound (I), in the formula, R₁, R₂, and R₃ are the same as or different from each other, and each represents lower alkyl, lower alkenyl, lower alkynyl, aralkyl, araryl (alkyl aryl), phenyl, or a hydrogen atom. Examples of such lower alkyl and alkyl portions of aralkyl and araryl include linear or branched C1-6 alkyl, and more specific examples thereof include methyl, ethyl, propyl, isopropyl, butyl, sec-butyl, tert-butyl, pentyl, isopentyl, neopentyl, and hexyl. In particular, methyl or ethyl is preferable.

Examples of lower alkenyl include linear or branched C2-6 alkenyl and more specific examples thereof include vinyl, allyl, 1-propenyl methacryl, crotyl, 1-butenyl, 3-butenyl, 2-pentenyl, 4-pentenyl, 2-hexenyl, and 5-hexenyl.

Examples of lower alkynyl include linear or branched C2-6 alkynyl and more specific examples thereof include ethynyl, propynyl, butynyl, pentinyl, and hexynyl.

Examples of aralkyl include C7-15 aralkyl and more specific examples thereof include benzyl, phenethyl, benzhydryl, and naphthylmethyl.

Examples of an aryl portion of araryl include C6-14 aryl and more specific examples thereof include phenyl, naphthyl, and anthryl. Accordingly, examples of araryl include methyl phenyl and ethyl phenyl.

PQQ (that is, a compound represented by general formula (I) above wherein R₁, R₂, and R₃ are all hydrogen atoms) can be produced by an organic chemical method (e.g., J. Am. Chem. Soc., 103, 5599-5600 (1981)) and a fermentation method. For example, PQQ can be produced by a method for producing pyrroloquinoline quinone (JP Patent Publication (Kokai) No. 1-218597 A (1989)), which comprises culturing a bacterium capable of assimilating methanol and producing pyrroloquinoline quinone in a culture medium comprising methanol as a carbon source in which the concentration of an iron compound is controlled.

Regarding a method for producing an ester body of PQQ represented by compound (I), such ester body can be synthesized from PQQ via an esterification reaction according to a conventional method.

A triester body of PQQ can be easily synthesized by a method that involves reacting PQQ or a salt thereof with alcohols under acidic conditions (e.g., JP Patent Publication (Kokai) No. 3-123781 A (1991) and JP Patent Publication (Kokai) No. 3-145492 A (1991)) or a method that involves reacting PQQ or a salt thereof with an alkyl halide, an alkenyl halide, an alkynyl halide, an aralkyl halide, an araryl halide, or the like in the presence of a base, for example. Moreover, the triester body of PQQ obtained by the above methods is partially hydrolyzed under acidic or basic conditions, so that a monoester body or a diester body can be obtained.

The thus obtained compound (I) can be separated and purified from a reaction solution by a general method such as column chromatography, recrystallization, and solvent extraction. Moreover, various means are employed for identification of the compound (I), such as elementary analysis, NMR spectrum, IR spectrum, and mass spectroscopy.

Examples of a salt of the compound (I) include alkali metal salts such as a sodium salt and a potassium salt, alkaline-earth metal salts such as a magnesium salt and a calcium salt, organic amine salts such as ammonium, triethanolamine, and triethylamine, and basic amino acid salts such as lysine and arginine.

The hypertension-ameliorating agent of the present invention can be a formulation comprising, as an active ingredient, the compound (I) or a salt thereof alone, a formulation comprising, as an active ingredient, a mixture thereof, or a formulation comprising a mixture of the compound (I) or a salt thereof with active ingredients for other arbitrary therapies. Such formulation is produced by mixing active ingredients with one or more types of pharmacologically acceptable carrier according to any method known in the technical field of galenical pharmacy.

The route of administration of the formulation that is the most effective for treatment is desirably used. Examples of such route of administration include oral administration and parenteral administration such as intravenous, intraperitoneal, or intradermal administration. Oral administration is preferable herein.

Examples of dosage forms that may be used for administration include: oral preparations such as tablets, powders, fine granules, pills, suspensions, emulsions, infusions, decoctions, capsules, syrups, liquids, elixirs, extracts, tinctura, and fluid extracts; and parenteral preparations such as injections, infusions, creams, and suppositories. In particular, oral preparations are adequately used.

Upon formulation of an oral preparation, an additive such as an excipient, a binder, a disintegrating agent, a lubricant, a dispersant, a suspension, an emulsifier, a diluent, a buffering agent, an antioxidant, or a microbial inhibitor can be used.

Furthermore, tablets, powders, fine granules, or the like, which are appropriate for oral administration, for example, can be formulated by adding: a saccharide such as lactose, saccharose, glucose, sucrose, mannitol, and sorbitol; starch such as potato, wheat, and corn; a mineral such as calcium carbonate, calcium sulfate, sodium hydrogencarbonate, and sodium chloride; an excipient such as crystalline cellulose and powdered plants (e.g., powdered glycyrrhiza, and powdered gentian); a disintegrating agent such as starch, agar, gelatin powder, crystalline cellulose, carmellose sodium, carmellose calcium, calcium carbonate, sodium hydrogencarbonate, and sodium alginate; a lubricant such as magnesium stearate, talc, hydrogenated vegetable oil, Macrogol, and silicone oil; a binder such as polyvinyl alcohol, hydroxypropyl cellulose, methyl cellulose, ethyl cellulose, carmellose, gelatin, and a starch paste solution; a surfactant such as a fatty acid ester; and a plasticizer such as glycerin, for example.

When the dosage form is a liquid preparation such as a syrup, formulation can be carried out by adding water, saccharides such as sucrose, sorbitol, and fructose, glycols such as polyethylene glycol and propylene glycol, oils such as sesame oil, olive oil, and soybean oil, antiseptics such as p-hydroxybenzoic acid esters, paraoxybenzoate derivatives such as methyl parahydroxybenzoate, preservatives such as sodium benzoate, flavors such as a strawberry flavor and peppermint, and the like.

Moreover, food additives that are generally used for foods or beverages may be added to formulations appropriate for oral administration. Examples of such additives include sweet-tasting substances, coloring agents, preservatives, thickening and stabilizing agents, antioxidants, color formers, bleaching agents, fungicides, gum bases, bitter-tasting substances, enzymes, brighteners, acidifiers, seasonings, emulsifiers, fortifier dietary supplements, additives for production, perfumes, and spice extracts. Formulations appropriate for oral administration may be directly used or used in the form of powdered foods, sheet-shaped foods, bottled foods, canned foods, retort-packed foods, capsulated foods, tablet foods, liquid foods, drinkable preparations, or the like as foods or beverages such as health foods, functional foods, nutritional supplements, or specified health foods for amelioration of hypertension.

For example, an injection appropriate for parenteral administration comprises a sterile aqueous agent that is preferably isotonic with the blood of a recipient and comprises the compound (I) or a salt thereof. For example, in the case of such injection, a solution for injection is prepared using a carrier or the like comprising a salt solution, a glucose solution, or a mixture of a salt solution and a glucose solution.

Furthermore, to these parenteral preparations, one or more types of auxiliary ingredient selected from among the aforementioned examples of oral preparations, such as diluents, antiseptics, flavors, excipients, disintegrating agents, lubricants, binders, surfactants, plasticizers, and the like can be added.

The dose and the frequency of administration of the formulation of the present invention differ depending on the route of administration, age and body weight of a patient, and characteristics or severity of symptoms to be treated. In general, the compound (I) or a salt thereof is administered once a day or several separate times a day for an adult so that the dose generally ranges from 0.5 mg to 10000 mg, preferably ranges from 0.5 mg to 5000 mg, and more preferably ranges from 5 mg to 1000 mg per day for an adult.

The period of administration is not particularly limited. It generally ranges from 1 day to 1 year and preferably ranges from 2 weeks to 3 months.

In addition, the formulation of the present invention can be used not only for humans, but also for animals other than humans (hereinafter, abbreviated as "non-human animal(s)"). Examples of non-human animals include animals other than humans, such as mammals, birds, reptiles, amphibians, and fishes.

The dose for administration to a non-human animal differs depending on the age and the type of the animal and the characteristics or severity of symptoms. In general, the compound (I) or a salt thereof is administered once a day or several separate times a day so that the dose generally ranges from 0.01 mg to 200 mg, preferably ranges from 0.1 mg to 100 mg, and more preferably ranges from 1 mg to 20 mg per kg of body weight per day.

The period of administration is not particularly limited. It generally ranges from 1 day to 1 year and preferably ranges from 2 weeks to 3 months.

### Examples

Hereinafter, the Test example in which hypertension-ameliorating effects of the compound (I) were examined is described.

### (Test example)

The rats used were spontaneously hypertensive rats (SHRs). The systolic blood pressure levels of 18 male SHR/Izm rats (10 week olds; Japan SLC, Inc.) were measured on Day 1 of administration. The rats were divided into 3 groups of 6 rats in a manner such that the mean values of the groups were almost equivalent to one another. The groups were referred to as the 1^{st} to the 3^{rd} groups.

A 0.5 w/v% methylcellulose aqueous solution was orally administered to the rats in the 1^{st} group. A 0.5 w/v% methylcellulose aqueous solution containing pyrroloquinoline quinone disodium salt (hereinafter, referred to as PQQ disodium salt; Mitsubishi Gas Chemical Company, Inc.) suspended therein (1 mg/mL) was orally administered to the rats in the 2^{nd} group. A 0.5 w/v% methylcellulose aqueous solution containing PQQ disodium salt suspended therein (4 mg/mL) was orally administered to the rats in the 3^{rd} group. Oral administration was carried out in a volume of 5 mL/kg once daily for 29 days. The single dose of PQQ disodium salt was 5 mg/kg in the 2^{nd} group and the same was 20 mg/kg in the 3^{rd} group. On Days 1, 8, 15, 22, and 29 of administration, systolic blood pressure was measured before administration (24 hours after administration on the previous day) and 4 hours after administration of the PQQ disodium salt. Systolic blood pressure was measured by the tail-cuff method with the use of an unheated non-invasive blood pressure monitor for mice and rats (MK-2000, Muromachi Kikai Co., Ltd.). Measurement was carried out 5 times per instance. The mean value of three systolic blood pressure values obtained by excluding the highest value and the lowest value was determined to be the value for an individual rat.

The value for each group was expressed as the mean value ± standard error (n = 6). A statistically significant difference was obtained at each measurement point by carrying out one-way analysis of variance. When the significance was confirmed by one-way analysis of variance, a parametric Dunnett's multiple comparison test was carried so that a significant difference between the 2^{nd} and the 1^{st} group and between the 3^{rd} group and the 1^{st} group were obtained. In addition, in a case in which there was a significance level (p value) of less than 0.05, it was determined that there was a significant difference.

Table 1 shows systolic blood pressure results obtained before administration.

**[Table 1]**

| | Systolic blood pressure (mmHg) | | | | |
|---|---|---|---|---|---|
| | Day 1 | Day 8 | Day 15 | Day 22 | Day 29 |
| The 1^{st} group | 172 ± 4 | 181 ± 4 | 191 ± 4 | 195 ± 3 | 195 ± 3 |
| The 2^{nd} group | 172 ± 3 | 177 ± 3 | 185 ± 3 | 189 ± 3 | 190 ± 1 |
| The 3^{rd} group | 172 ± 3 | 174 ± 2 | 182 ± 3 | 184 ± 3 | 186 ± 3 |

As shown in table 1, the systolic blood pressure value for the 1^{st} group gradually increased over the course of the test period, indicating the advancement of hypertension. On the other hand, the systolic blood pressure value for the 2^{nd} group was lower than that for the 1^{st} group from Day 8 to the end of the test period. Further, the systolic blood pressure value for the 3^{rd} group was lower than that for the 2^{nd} group.

Table 2 shows systolic blood pressure results obtained 4 hours after the administration of PQQ disodium salt.

**[Table 2]**

| | Systolic blood pressure (mmHg) | | | | |
|---|---|---|---|---|---|
| | Day 1 | Day 8 | Day 15 | Day 22 | Day 29 |
| The 1^{st} group | 173 ± 4 | 181 ± 5 | 192 ± 4 | 195 ± 3 | 194 ± 2 |
| The 2^{nd} group | 172 ± 4 | 175 ± 3 | 184 ± 3 | 189 ± 3 | 190 ± 1 |
| The 3^{rd} group | 171 ± 3 | 171 ± 2 | 180 ± 3 | 184 ± 3 | 184 ± 3* |

| | | | | | |
|---|---|---|---|---|---|
| * : p < 0.05 relative to the value for the 1^{st} group | | | | | |

As shown in table 2, the systolic blood pressure value for the 2^{nd} group was lower than that for the 1^{st} group from Day 8 to the end of the test period. Further, the systolic blood pressure value for the 3^{rd} group was lower than that for the 2^{nd} group. The value for the 3^{rd} group was significantly lower than that for the 1^{st} group 4 hours after administration of PQQ disodium salt on Day 29.

As is apparent from the above results, blood pressure-reducing effects, namely, hypertension-ameliorating effects, were observed in hypertension rats to which PQQ disodium salt had been administered.

Next, with reference to the Examples in which formulation examples of the composition of the present invention are described, the present invention is further specifically described. However, the present invention is not limited to such examples.

### (Example 1)

A soft drink for amelioration of hypertension (in a volume corresponding to 10 bottles) is prepared in accordance with the formulation listed in table 3.

**[Table 3]**

| Composition | Content |
|---|---|
| PQQ disodium salt | 100 mg |
| Vitamin C | 1 g |
| Vitamin B 1 | 5 mg |
| Vitamin B2 | 10 mg |
| Vitamin B6 | 25 mg |
| Liquid sugar | 150 g |
| Citric acid | 3 g |
| Perfume | 1 g |

Water is added to a resultant to a volume of 1000 mL.

### (Example 2)

A tea drink for amelioration of hypertension (1000 mL) is prepared in accordance with the formulation listed in table 4.

**[Table 4]**

| Composition | Content |
|---|---|
| PQQ dimethyl ester | 100 mg |
| Tea leaves | 15 g |

The resultant is eluted with water (1000 mL).

### (Example 3)

Chewing gum (in an amount corresponding to 30 pieces) for amelioration of hypertension is prepared in accordance with the formulation listed in table 5.

**[Table 5]**

| Composition | Content |
|---|---|
| PQQ trimethyl ester | 0.1 g |
| Gum base | 25 g |
| Sugar | 63 g |
| Sugar syrup | 10 g |
| Perfume | 1 g |

### (Example 4)

Candy (in an amount corresponding to 20 pieces) for amelioration of hypertension is prepared in accordance with the formulation listed in table 6.

**[Table 6]**

| Composition | Content |
|---|---|
| PQQ disodium salt | 0.1 g |
| Sugar | 80 g |
| Sugar syrup | 20 g |
| Perfume | 0.1 g |

### (Example 5)

Tablets for amelioration of hypertension (200 mg each) are prepared by a conventional method in accordance with the formulation listed in table 7.

**[Table 7]**

| Composition | Content |
|---|---|
| PQQ disodium salt | 5 mg |
| Lactose | 120 mg |
| Cornstarch | 45 mg |
| Synthetic aluminium silicate | 12 mg |
| Carboxy methylcellulose.calcium | 15 mg |
| Magnesium stearate | 3 mg |

### (Example 6)

A powder for amelioration of hypertension (550 mg for each package) is prepared by a conventional method in accordance with the formulation listed in table 8.

**[Table 8]**

| Composition | Content |
|---|---|
| PQQ diethyl ester | 5 mg |
| Lactose | 330 mg |
| Cornstarch | 215 mg |

### (Example 7)

A hard capsule for amelioration of hypertension (160 mg for each capsule) is prepared in accordance with the formulation listed in table 9.

**[Table 9]**

| Composition | Content |
|---|---|
| PQQ monoallyl ester | 5 mg |
| Lactose | 90 mg |
| Cornstarch | 45 mg |
| Hydroxypropyl cellulose | 20 mg |

Lactose (90 mg) and cornstarch (45 mg) are added to PQQ monoallyl ester (5 mg), followed by mixing. An aqueous solution containing hydroxypropyl cellulose (20 mg) is added thereto, followed by kneading. Subsequently, granules are prepared by a conventional method with the use of an extrusion granulator. Gelatin hard capsules are filled with the granules such that a hard capsule preparation is prepared.

### (Example 8)

A soft capsule for amelioration of hypertension (170 mg for each capsule) is prepared in accordance with the formulation listed in table 10.

**[Table 10]**

| Composition | Content |
|---|---|
| PQQ disodium salt | 5 mg |
| Soybean oil | 165 mg |

PQQ disodium salt (5 mg) is added to soybean oil (165 mg), followed by mixing. Subsequently, soft capsules are filled with the resultant by a conventional method with the use of an automatic rotary die molding machine such that a soft capsule is prepared.

### Industrial Applicability

The present invention provides a hypertension-ameliorating agent comprising pyrroloquinoline quinone or an ester or salt thereof as an active ingredient.

All publications, patents, and patent applications cited herein are incorporated herein by reference in their entirety.

## Claims

1. A hypertension-ameliorating agent, which comprises a compound represented by general formula (I) 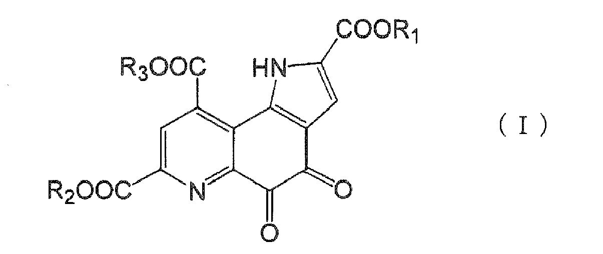 (wherein R₁, R₂, and R₃ are the same as or different from each other, and each represents lower alkyl, lower alkenyl, lower alkynyl, aralkyl, araryl, phenyl, or a hydrogen atom) or a salt thereof as an active ingredient.

2. A method for improving hypertension, which comprises administering an effective amount of the compound represented by general formula (I) according to claim 1 or a salt thereof to a subject in need thereof.

3. Use of the compound represented by general formula (I) according to claim 1 or a salt thereof for the manufacture of a hypertension-ameliorating agent.
